# EUROPEAN PATENT APPLICATION

(11) **EP 3 842 413 A1**
(43) Date of publication of application: **30.06.2021**
(21) Application number: 19852401.9
(22) Date of filing: 23.08.2019
(51) Int. Cl.: C07C 231/22, C07C 233/20

(54) **METHOD FOR STABILIZING SANSHOOL COMPOUND**

(30) Priority: 23.08.2018 JP 2018156314
(71) Applicant: Yamamoto, Yoshie, Kainan-shi, Wakayama 642-0024 (JP); Wakayama University, Wakayama-shi, Wakayama 640-8510 (JP)
(72) Inventor: MITANI, Takahiko, Wakayama-shi, Wakayama 640-8510 (JP); TSUCHIDA, Takashi, Kainan-shi, Wakayama 642-0024 (JP)
(74) Representative: Vossius & Partner Patentanwälte Rechtsanwälte mbB
(86) International application number: PCT/JP2019/034878
(87) International publication number: WO 2020/040315

(57) **Abstract**

A method for stabilizing a sanshool compound is provided. The method for stabilizing a sanshool compound comprises a mixing step in which the sanshool compound is mixed with an antioxidant in a liquid, the liquid comprising water and/or a water-soluble organic solvent and the mixing step being conducted under the conditions of a pH of 6 or higher.

## Description

### [Technical field]

The present invention relates to a method for stabilizing sanshool, a sanshool-containing composition, and a method for producing the same.

### [Background of the Invention]

It is known that sansho (Zanthoxylum piperitum) contains a pungent ingredient that can cause numbness, and that the numbing effect of this pungent ingredient is caused by amide derivatives such as hydroxy sanshool contained in a sansho plant (Patent Document 1). Further, an extract extracted from a sansho plant is also known to be useful as a food, a cosmetic preparation, a pharmaceutical preparation or the like (Patent Documents 1 and 2).

### [Prior art reference]

### [Patent document]

[Patent document 1] JP 2013-103901A
[Patent document 2] JP 2001-354958A

### [Summary of the invention]

### [Problem to be solved by the invention]

Incidentally, when sansho plants are used as a component of a product such as a food product, a cosmetic preparation, a pharmaceutical preparation or the like, a fruit or a pericarp of sansho plants may be powdered or extracted and purified. The flavor, taste, scent, and color tone of sansho fruits and pericarps are relatively stable in a dried state, but a powdery form of sansho or a sansho extract tends to be deteriorated when exposed to air or light, or when stored at room temperature. If the powdery product or extract of sansho plants deteriorate, then the quality of the product containing sansho plants may deteriorate as well. Therefore, a method for inhibiting degradation of the powdery product or the extract of sansho plants is in demand.

The object of the present invention is to provide a method for stabilizing sanshool, a sanshool-containing composition, and a method for producing the same.

### [Means for solving the problem]

The present invention provides a method for stabilizing sanshool, a sanshool-containing composition, and a method for producing the same.
[1] A method for stabilizing sanshool comprising a mixing step of mixing an amount of sanshool with an amount of antioxidant in a liquid.
[2] In the method for stabilizing sanshool, the liquid contains at least one selected from the group consisting of water and a water-soluble organic solvent, and the mixing step is performed under the condition of pH 6 or more.
[3] In the method for stabilizing sanshool, the antioxidant is one or more selected from the group consisting of dihydroxybenzoic acid or salt thereof or ester thereof, trihydroxybenzoic acid or salt thereof or ester thereof, hydroxycinnamic acid or salt thereof, ascorbic acid or salt thereof or ester thereof and tocopherol.
[4] A sanshool-containing composition comprising an amount of sanshool, an amount of antioxidant and a liquid, wherein the liquid includes at least one selected from a group consisting of water and a water-soluble organic solvent.
[5] In the sanshool-containing composition, the antioxidant is one or more selected from the group consisting of dihydroxybenzoic acid or salt thereof or ester thereof, trihydroxybenzoic acid or salt thereof or ester thereof, hydroxycinnamic acid or salt thereof, ascorbic acid or salt thereof or ester thereof and tocopherol.
[6] A method for producing a sanshool-containing composition including a mixing step of mixing sanshool and an antioxidant in a liquid, wherein the liquid contains at least one selected from the group consisting of water and a water-soluble organic solvent, wherein the mixing step is performed under the condition of pH 6 or more.

### [Effect of the Invention]

According to the present invention, a method for stabilizing sanshool capable of stabilizing sanshool, a sanshool-containing composition, and a method for producing the same are provided.

### [Embodiments of the invention]

### (A method for stabilizing sanshool)

The method for stabilizing sanshool of the present embodiment includes a mixing step of mixing an amount of sanshool with an amount of antioxidants in a liquid. According to this stabilization method, sanshool can be easily stored for a long period of time. When it is used in various products such as foods, supplements, beverages, pharmaceutical preparations, quasi-drugs, cosmetic preparations, feeds, detergents, commodities, and the like, sanshool may be possible to present in a stable state, so that it is expected to prolong the life of the products.

### (Sanshool)

Sanshool may be extracted from sansho plants (Zanthoxylium peperitum), or it may be synthesized. Examples of the sanshool of the present embodiment may include α-sanshool, β-sanshool, γ-sanshool, δ-sanshool, hydroxy-α-sanshool, hydroxy-β-sanshool, and hydroxy-γ-sanshool. Among these, hydroxy-α-sanshool or α-sanshool which is contained in a large amount in sansho plants is preferable.

Sanshool is known not only as a pungent ingredient in foods, but also known as ingredients in supplements or medicines such as salty taste enhancers, gastrointestinal motility improving agents namely gastroparesis or postsurgical ileus improving agents, antioxidants, anesthetic agents, memory promoting agents, fat-burning agents and the like. When sanshool becomes stabilized by the stabilizing method of sanshool of the present embodiment, various products containing the stabilized sanshool can be expected to have an improved shelf time.

Sanshool may be included in a sansho extract extracted from sansho plants (hereinafter sometimes simply referred to as "sansho extract"). The sansho extract is defined as an extract extracted from a part forming a sansho plant and contains at least sanshool. The sansho extract may contain other extracts than sanshool extracted from a sansho plant. The sansho extract may be present in a solvent used for extracting an extract of a sansho plant such as sanshool, and may be present in an extract from which at least a part of the solvent is removed, or may compose a dried product obtained by removing a solvent from the wet sansho extract by drying.

There is no particular limitation in sansho varieties that can be used for obtaining the sansho extract, as long as it is a plant included in Zanthoxylum genus. Specific examples of the sansho plants may include sansho (Zanthoxylum piperitum DC), asakurasansho (Zanthoxylum piperitum DC.var.inerme Makino), yamaasakurasansho (Zanthoxylum piperium DC. forma brevispinosum Makino), inusansho (Zanthoxylum schinifolium Sieb.et Zucc.), kahokusansho (Zanthoxylum bungeanum Maxim), fuyusansho (Zanthoxylum armatum var.subtrifoliatum), and terihasansho (Zanthoxylum nitidum). Among these, sansho (Zanthoxylum piperitum DC) and asakurasansho (Zanthoxylum piperitum DC.var.inerme Makino), which are commonly used in foods and excel in quality and safety, are more preferable. Above all, the variety called "Budosansho" which is a special product from the Wakayama prefecture is even more preferable.

The parts of sansho plants used for obtaining the sansho extract may be any of fruit, pericarp, endocarp, seed, leaf, trunk, branch, root, stem, and the like of sansho plants. From the viewpoint of collection efficiency and workability, it is preferable to use fruit, pericarp, endocarp, and leaf; and more preferably, fruit, pericarp, and endocarp of sansho plants; and more preferably, fruit and pericarp of sansho plants.

There is no particular limitation on the extraction method for obtaining an sansho extract from a part of sansho plants, as long as it is a method which is conventionally known. Examples thereof include a compression method, a steam distillation method, a solvent extraction method, a supercritical extraction method, a carbon dioxide extraction method, and an enfleurage method. Among these, a solvent extraction method, in which sanshool may be efficiently extracted, is preferable.

In the solvent extraction method, a part of sansho plants may optionally be subjected to a pretreatment process such as drying, cutting, crushing, grinding, or the like, and then extracted by using an organic solvent, water, or a liquid mixture of water and an organic solvent acting as an extraction solvent. Examples of the organic solvent may include an organic solvent to be described later.

When sanshool is obtained through the process of synthesis, the sanshool may be synthesized according to any known synthetic method.

### (Antioxidant).

An antioxidant is a substance which is generally considered to have an antioxidant function. Particularly safe antioxidants which can be used in foods and pharmaceutical preparations are preferred. The antioxidant in the disclosed embodiments may be water-soluble or fat-soluble.

Examples of a water-soluble antioxidant may include dihydroxybenzoic acid such as protocatechuic acid or salts thereof; trihydroxy benzoic acid such as gallic acid or salt thereof; hydroxycinnamic acids such as caffeic acid and ferulic acid or salt of these examples; ascorbic acids such as ascorbic acid and erythorbic acid or salt of these examples; and thiols such as thiourea, cysteine and glutathione; and lipoic acids such as α-lipoic acid, dihydrolipoic acid, lipoamide or dihydrolipoamide. Examples of the salt of these acids may include alkali metal salt or an alkaline earth metal, and examples thereof include sodium salt, potassium salt, magnesium salt and calcium salt.

Examples of the fat-soluble antioxidant include esters of dihydroxybenzoic acid; esters of trihydroxybenzoic acid; tocopherols such as α-tocopherol or β-tocopherol; and flavonoids such as quercetin or naringenin. Examples of the esters of the above-mentioned acids include those having an alkyl ester group having 1 to 5 carbon atoms.

One or more kinds of antioxidant may be used to be mixed with sanshool, and two or more antioxidants may be considered as option. When two or more antioxidants are used, it may be preferred to use a combination of a water-soluble antioxidant and a fat-soluble antioxidant.

### (Mixing process)

In the method of stabilizing sanshool, the mixing step is a step of mixing an amount of sanshool with an amount antioxidant in a liquid. The sanshool and the antioxidant may be dissolved in the liquid or dispersed in the liquid. The liquid used in the mixing step is not particularly limited. Examples thereof include an organic solvent, water, and a liquid mixture of water and an organic solvent. In the case of stabilizing sanshool contained in a sansho extract, the liquid used in the mixing step may be the extraction solvent used for extracting the sansho extract. To stabilize the sanshool obtained by synthesis, the liquid used in the mixing step may be the solvent used in the synthesis, for example, the solvent used for purification.

Examples of the organic solvent used in the mixing step as a liquid may include alcohols having 1 to 4 carbon atoms such as ethanol, methanol, 1-propanol, 2-propanol, 1-butanol, 2-butanol; ethers such as diethyl ether, tetrahydro furan; esters such as ethyl acetate; ketones such as acetone; nitriles such as acetonitrile; aromatic hydrocarbons such as benzene and toluene; halogenated aliphatic hydrocarbons such as methylene chloride and chloroform; and hexanes; and mixtures thereof. Among these solvents, preferred is an alcohol having 1 to 4 carbon atoms, esters, acetone, halogenated aliphatic hydrocarbons, or a liquid mixture of one or more of water-soluble organic solvents (to be described later) among these organic solvents and water, and more preferably an alcohol having 1 to 4 carbon atoms, acetone, ethyl acetate, acetonitrile, chloroform and a liquid mixture of one or more of water-soluble organic solvents among these organic solvents and water, and further more preferably ethanol, methanol, 2-propanol, acetone, ethyl acetate, acetonitrile, chloroform, and a liquid mixture of one or more of water-soluble organic solvents among these organic solvents and water.

In addition, when stabilized sanshool that has been stabilized through the stabilization method of sanshool are used for foods and pharmaceutical preparations, it is preferable to use a safe solvent which can be used for a food and a pharmaceutical preparation such as ethanol or 1-propanol.

Sanshool is easily degraded by decomposition such as by oxidation and by hydrolysis. For this reason, it is preferred that the mixing step of sanshool and an antioxidant is carried out as early as possible after the sanshool has been extracted or after the process of synthesizing the sanshool has been completed. An amount of sanshool and an amount of antioxidant may be mixed preferably in a liquid immediately after extraction or synthesis. For example, in the production of a pharmaceutical preparation or a food supplement containing sanshool, the sanshool and the antioxidant are mixed beforehand to stabilize the sanshool. Then the stabilized sanshool and a material which forms a pharmaceutical preparation or a supplement (a product for applying the sanshool) are mixed. Thus, it is possible for the sanshool present in a stabilized condition in these products, so that it is expected to prolong the life of these products.

The mixing step of mixing an amount of sanshool with an amount of antioxidants in a liquid may be performed in one step, or may be performed in two or more steps. The mixing step of the sanshool that have been extracted from the sansho plant with an antioxidant may be performed in two steps, for example, as follows: in a 1st mixing step, an amount of antioxidant is added to an amount of extract obtained by extracting sanshool from sansho plants; thereafter, the extract obtained in the 1st mixing step by adding an antioxidant is concentrated or dried to obtain the sanshool containing an antioxidant (hereinafter, "antioxidant-containing sanshool"). In a 2nd mixing step, the antioxidant-containing sanshool and an amount of antioxidant are then mixed in a liquid. The antioxidant used in the 1st mixing step and the antioxidant used in the 2nd mixing step may be the same or different. In addition, for the sanshool obtained by synthesis, a mixing step may be performed in two or more steps as described above. By carrying out the mixing step in two or more steps as described above, it is possible not only to stabilize sanshool immediately after obtaining sanshool (the 1st mixing step), but also to stabilize sanshool that has been incorporated into various products such as foods, supplements, beverages, pharmaceutical preparations, quasi-drugs, cosmetic preparations, feeds, detergents, commodities, and the like, by adding an antioxidant to sanshool (antioxidants-containing sanshool), by which further stabilization effect of sanshool can be expected.

When the liquid contains at least one selected from the group consisting of water and a water-soluble organic solvent, the mixing step is preferably performed under the condition such that the pH of the liquid reaches 6 or more, yet more preferably pH 6.5 or higher, and still more preferably pH 7.0 or higher. The upper limit of pH is not particularly limited, but is usually 14 or less, preferably 12 or less, and may be 10 or less, or 9 or less. When the performance of the mixing step under the condition of pH 6 or more has resulted in a liquid containing at least one selected from the group consisting of water and a water-soluble organic solvent, it is possible to effectively stabilize sanshool. In this case, the antioxidant may be a water-soluble antioxidant, a fat-soluble antioxidant, or a combination thereof. In this specification, a water-soluble organic solvent refers to an organic solvent which dissolves at least 5% by weight in water. Examples of the water-soluble organic solvents include an alcohol having 1 to 4 carbon atoms, an ether, an ester, a ketone, and a nitrile.

The pH of the liquid in the mixing step may be adjusted according to the type of the antioxidant, or may be adjusted by using a buffer, an alkaline agent, or the like. Examples of the alkaline agent include potassium carbonate, sodium carbonate, sodium hydrogen carbonate, potassium salt or sodium salt of phosphoric acids. In the mixing step, there is no particular limitation of sequence as long as the sanshool and the antioxidant can be mixed in the liquid, and the antioxidant may be added to a mixture containing the liquid and sanshool (mixture A). An amount of sanshool may be added to a mixture containing the liquid and the antioxidant (mixture B), or the mixture A and the mixture B may be mixed, or an amount of sanshool and an amount of antioxidant may be added and mixed in the liquid. In the mixing procedure described above, the pH may be maintained at 6 or more in the mixing step, the pH may be adjusted while mixing the mixture A, the mixture B, and the liquid, and the pH can be measured while mixing the sanshool with the antioxidant and an alkaline agent may be added to keep the pH at a number equal to or more than 6.

It may be presumed that sanshool is easily hydrolyzed under acidic conditions. Depending on the kind and the dosage of sanshool that is added, the conditions under which the amount of sanshool is added, etc., the antioxidant of this embodiment can incline to acidic conditions, when the antioxidant of this embodiment is mixed with the amount of sanshool. For this reason, in the method for stabilizing sanshool, when a liquid containing at least one selected from the group consisting of water and a water-soluble organic solvent is used in the mixing step, the mixing of sanshool with an amount of antioxidant under the condition of pH 6 or more suppresses the oxidation process of sanshool by the antioxidant, and also suppresses the process of hydrolysis of sanshool, so that sanshool can be stabilized.

When a fat-soluble organic solvent such as an aromatic hydrocarbon, a halogenated aliphatic hydrocarbon, or hexane is included as a liquid in the mixing step, a fat-soluble antioxidant is preferably used.

In application of sanshool to various products (foods, supplements, beverages, pharmaceutical preparations, quasi-drugs, cosmetic preparations, feeds, detergents, commodities, etc.), a liquid containing water is often used. Therefore, in the 1st mixing step, the mixing step may be performed by using an optional liquid and an optional antioxidant, and in the 2nd mixing step, an amount of antioxidant and an amount of sanshool (an antioxidant-containing sanshool) may be mixed with a liquid containing water, for example.

The dosage of the antioxidant added in the mixing step may depend on the type of the antioxidant, but in general, 0.2 mol or more per 1 mol of the sanshool is preferable, more preferably 0.5 mol or more, and may be 1 mol or more, may be 5 mol or more, may be 10 mol or more, may be 15 mol or more, may be 30 mol or less, may be 25 mol or less, or may be 20 mol or less.

### (Sanshool-containing composition)

The composition of the present invention comprises sanshool, an antioxidant, and a liquid, wherein the pH is 6 or more, and the liquid contains at least one selected from the group consisting of water and a water-soluble organic solvent. The sanshool, the antioxidant, and the liquid are as described above. The sanshool-containing composition may include at least one selected from the group consisting of water and a water-soluble organic solvent, and may be a mixture of water and a water-soluble organic solvent. The antioxidant may be a water-soluble antioxidant, a fat-soluble antioxidant, or may comprise 2 or more antioxidants. The sanshool-containing composition may be in a state in which the sanshool and the antioxidant are present in the liquid, and may be in a dispersed state in which the sanshool and the antioxidant are dispersed, or may be in an emulsified state in which an emulsifying agent has been added.

It is more preferable that the pH of the sanshool-containing composition is 6 or more, and it is even more preferable if the pH is 6.5 or more, and more preferable 7.0 or more. Further, the upper limit of the pH of the sanshool-containing composition is not particularly limited, but is usually 14 or less, preferably 12 or less, and may be 10 or less, and may be 9 or less. When the pH of the sanshool-containing composition is 6 or more, it is possible to effectively stabilize the sanshool.

The content of the antioxidant contained in the sanshool containing composition depends on the type of the antioxidant, but in general, 0.2 mol or more per 1 mol of the sanshool is preferable, more preferably 0.5 mol or more, and may be 1 mol or more, may be 5 mol or more, may be 10 mol or more, may be 15 mol or more, may be 30 mol or less, may be 25 mol or less, or may be 20 mol or less. If the content of the antioxidant is too small compared to sanshool, it tends to be difficult to stabilize sanshool in a sanshool-containing composition.

Although the sanshool-containing composition may contain a liquid, the sanshool-containing composition may be dried to remove the liquid to form a particulate product or a powdery product, which may be used in various products. As a method of removing the liquid from a sanshool-containing composition, a known drying means such as spray drying, freeze drying, and vacuum drying may be used.

The sanshool-containing composition may then be used, (a) as a liquid product, (b) as a granular product or a powdery product by drying to remove the liquid or (c) by dissolving the granular product or the powdery product in a liquid again, and is added to various products such as a food, a supplement, a beverage, a pharmaceutical preparation, a quasi-drug, a cosmetic preparation, or a feed product for feeding animals. By using the sanshool-containing composition, it is expected that the shelf life (shelf time) of these products can be improved because the sanshool can be present in a stable form in these products.

### (A method for producing a sanshool-containing composition)

The method for producing the sanshool-containing composition of the present embodiment includes a mixing step of mixing an amount of sanshool and an amount of antioxidant in a liquid, wherein the liquid includes at least one selected from the group consisting of water and a water-soluble organic solvent, and the mixing step is performed under the condition of pH 6 or more. The sanshool, the antioxidant, the liquid, and the mixing steps are as described above. The liquid used in the mixing step may include at least one selected from the group consisting of water and a water-soluble organic solvent, and may be a liquid mixture of water and a water-soluble organic solvent. The anti-oxidant may be a water-soluble antioxidant, or may be a fat-solvable antioxidant, or may contain two or more kinds of anti-oxidants.

The method of producing a sanshool-containing composition may include a drying step of removing at least a part of the liquid after the mixing step, and may include an emulsification step of adding an emulsifier to the composition to emulsify the composition. The drying steps may include a concentration step of removing a part of the liquid, or the sanshool-containing composition containing the liquid may be dried by spray drying, freeze drying, vacuum drying, or the like to obtain a particulate product or a powdery product.

The sanshool-containing composition obtained by the method for producing the above-mentioned sanshool-containing composition may be added to various products such as foods, supplements, beverages, pharmaceutical preparations, quasi-drugs, cosmetic preparations, and feeds. Using the sanshool-containing composition obtained by the production method mentioned above can improve the shelf life such as shelf time of the various products because sanshool is stably present in these products.

### [Examples]

Hereinafter, the present invention will be described in more detail by referring to Examples and Comparative Examples, but the present invention may not be limited these Examples.

### <High Performance Liquid Chromatography (HPLC) Analysis>

High performance liquid chromatography (HPLC) analysis was performed on reverse phase systems. An HPLC device used was LC-2010 (manufactured by Shimadzu Corporation), a pump used was LC-10 AdVp, a column oven used was CTO-10 ACvp, and a system controller used was SCL-10 Avp. A column was InertSustainSwift C18 4.6x250mm (GL Sciences). The column temperature was 40 degrees Celsius, and, as for a mobile phase, liquid A was 30% acetonitrile and liquid B was 80% acetonitrile while gradient conditions were 0 min B solution 0% → 35min B solution 45% → 50 min B solution 100% → 51 min B solution 0% → 55 min B solution 0%, and the detection wavelength was set at 270 nm, and the hydroxy-α-sanshool content was calculated from the peak area value of the hydroxy-α-sanshool.

### <Measurement of pH>

A pH measurement apparatus used was pH Meter F52, manufactured by HORIBA Co., Ltd..

### <Examples 1 to 31>

A fully-ripe sansho plant (variety name: Budosansho, product of the Wakayama prefecture, Japan) was dried, and the pericarp and seeds were separated by sieving through a sieve, and endocarps between pericarp and seed was collected. The endocarp was ground with a laboratory mill (LM-PLUS, manufactured by Osaka Chemical Co., Ltd.) and passed through a sieve having an opening of 250 µm to obtain the powdery product of endocarp of sansho plants having passed through the sieve. To 0.4 g of this powder, 8 mL of aqueous solution containing 50% by volume of ethanol as extraction solvent was added, then suspended, then shaken by a vortex mixer, sonicated, centrifuged, and then the supernatant was collected. The collected supernatant and the corresponding antioxidant shown in Table 1 were put into a Spitz tube and kept in an oil bath set at a temperature of 70 ° C. The content of hydroxy-α-sanshool in 1 ml of the supernatant was 2.5 µ mol. Further, the dosages of antioxidants added and the molar ratios of the antioxidants to 1 mol of hydroxy-α-sanshool are shown in Tables 1 and 2.

Before performing heat retention on the sample containing the supernatant and the antioxidant, the content of hydroxy-α-sanshool in the sample was measured by using HPLC. Further, the sample was kept warm in an oil bath, and the content of hydroxy-α-sanshool in the sample on the 9th day of heat retention was measured by HPLC. Using measurement results obtained by the HPLC measurement, the measured value obtained by HPLC measurement of the sample on day 0 (before heat retention in the oil bath had been performed) is set as 100. Then the ratio of the measured value obtained by HPLC measurement of the sample on the 9th day of the heat retention (the content of the hydroxy-α-sanshool on the 9th day of the heat retention / the content of the hydroxy-α-sanshool on the day 0) × 100 [%] was calculated, and the survival rate (%) was obtained. The results are shown in Table 1 and Table 2. For some Examples, the results of pH measurement performed prior to heat retention of the sample containing supernatant and an antioxidant in an oil bath are shown. In Examples 7 to 10 and 29 and 30, pH was adjusted by using sodium hydroxide. For the other Examples, no pH adjustment was made.

### <Comparative Example 1>

In the same manner as in Examples 1 to 31 except that no antioxidant was added, the amount of the sample before the oil bath heat retention and the content of the hydroxy-α-sanshool on the 9th day of the oil bath heat retention were measured by HPLC. The results are shown in Table 2. In Comparative Example 1, pH was not adjusted.

Protocatechuic acid, gallic acid, caffeic acid, and naringenin which were used in each Example and each Comparative Example were the products of Sigma-Aldrich, while ethyl gallate, α-tocopherol, sodium ascorbic acid, sodium erythorbate, ferulic acid, and quercetin were the products of Wako Pure Chemical Industries, Ltd. Sodium gallate was prepared by neutralizing gallic acid with NaOH.

**[Table 1]**

| | *Antioxidant* | pH | *Antioxidant* | | Survival ratio (%) |
|---|---|---|---|---|---|
| | | | Dosage (µmol/mL) | Molar ratio (*1) | |
| Example 1 | Protocatechuic acid | - | 1.625 | 0.65 | 57.3 |
| Example 2 | | 3.5 | 6.5 | 2.6 | 56.9 |
| Example 3 | Gallic acid | - | 0.73 | 0.29 | 68.8 |
| Example 4 | | 3.3 | 1.5 | 0.59 | 70.8 |
| Example 5 | | 3.2 | 2.9 | 1.18 | 63.5 |
| Example 6 | | 3.0 | 5.9 | 2.35 | 58.5 |
| Example 7 | | 6.9 | 5.9 | 2.35 | 89.4 |
| Example 8 | | 8.7 | 5.9 | 2.35 | 92.5 |
| Example 9 | | 10.0 | 5.9 | 2.35 | 93.6 |
| Example 10 | | 11.0 | 5.9 | 2.35 | 94.0 |
| Example 11 | Ethyl gallate | - | 0.65 | 0.26 | 72.0 |
| Example 12 | | 6.4 | 1.3 | 0.52 | 71.5 |
| Example 13 | | 6.3 | 2.6 | 1.04 | 70.9 |
| Example 14 | | 6.0 | 5.2 | 2.08 | 67.8 |
| Example 15 | Sodium gallate | - | 0.65 | 0.26 | 67.8 |
| Example 16 | | - | 1.3 | 0.52 | 75.8 |
| Example 17 | | - | 2.6 | 1.04 | 83.3 |
| Example 18 | | 7.8 | 5.2 | 2.08 | 87.2 |
| Example 19 | Arbutin | - | 0.92 | 0.368 | 55.7 |

| | | | | | |
|---|---|---|---|---|---|
| *1 The molar ratio of the antioxidant to hydroxy-alfa-sanshool | | | | | |

**[Table 2]**

| | *Antioxidant* | pH | *Antioxidant* | | Survival rate (%) |
|---|---|---|---|---|---|
| | | | Dosage (µmol/mL) | Molar ratio (*1) | |
| Example 20 | Caffeic acid | - | 1.4 | 0.56 | 58.9 |
| Example 21 | | 3.7 | 5.6 | 2.24 | 60.0 |
| Example 22 | Ferulic acid | - | 1.29 | 0.51 | 53.1 |
| Example 23 | Quercetin | - | 0.825 | 0.33 | 53.2 |
| Example 24 | Naringenin | - | 0.925 | 0.37 | 54.2 |
| Example 25 | alfa-tocopherol | 6.4 | 0.058 | 0.0232 | 53.1 |
| Example 26 | | 6.4 | 0.58 | 0.232 | 61.6 |
| Example 27 | | 6.4 | 5.8 | 2.32 | 63.8 |
| Example 28 | Sodium ascorbate | - | 6.25 | 2.5 | 54.0 |
| Example 29 | | 7.4 | 50 | 20 | 82.4 |
| Example 30 | | 9.0 | 50 | 20 | 84.0 |
| Example 31 | Sodium erythorbate | - | 0.63 | 0.25 | 58.8 |
| Comparative Example 1 | - | 6.4 | - | - | 49.7 |

| | | | | | |
|---|---|---|---|---|---|
| *1 The molar ratio of the antioxidant to hydroxy-alfa-sanshool | | | | | |

Note that, in the above Examples and Comparative Examples, since the content of other kinds of sanshool than hydroxy-α-sanshool in the sansho extract is very small compared to the content of hydroxy-α-sanshool, the content of hydroxy-α-sanshool can be approximated to the content of sanshool in the sansho extract. Further, a storing the sanshool for 9 days in an oil bath at a temperature of 70 °C as performed in the above Examples and Comparative Examples provides a suitable condition for an accelerated test for predicting the stability of the compound. In the accelerated test, storing the sanshool at a temperature of 70°C for 7 days can be regarded as equivalent to storing at a temperature of 25°C (room temperature) for 3 years, so that a storage treatment at 70 °C for 9 days performed in Examples and Comparative Examples is considered to correspond to a storage at the temperature of 25 °C for over 3 years.

### [Example 32]

The contents of the hydroxy-α-sanshool in the sample on day 8 of heat retention in the oil bath were measured by HPLC for the cases where as an antioxidant, (a) sodium gallate 0.65 µmol/ml was used, (b) sodium ascorbate 25 µmol/ml was used, and (c) sodium gallate 0.65µmol/mL and sodium ascorbate 25 µmol/ml were used, and the survival rate was measured in the same manner as in Examples 1 to 31.

As a result, it was confirmed that the survival rate of hydroxy-α-sanshool in the case of (c) is almost equal to the total amount of the survival rate of hydroxy-α-sanshool in the case of (a) and the survival rate of hydroxy-α-sanshool in the case of (b), and an additive effect among the antioxidants was confirmed.

### [Example 33]

The content of hydroxy-α-sanshool contained in the sample after performing heat retention for 8 days in the oil bath was measured by HPLC for the cases where as an antioxidant (b) sodium ascorbate 25 µmol/ml is used, (d) tocopherol 0.58µmol/ml is used, (e) sodium gallate 0.65µmol/ml and sodium ascorbate 25 µmol/ml were used, and the survival rate was measured in the same manner as in Example 32,

As a result, the survival rate of hydroxy-α-sanshool (e) above was confirmed substantially equal to the total amount of the survival rate of hydroxy-α-sanshool of (b) and the survival rate of hydroxy-α-sanshool of (d). Namely, an additive effect among the antioxidants was confirmed.

### [Examples 34 to 37]

In the same manner as in Examples 1 to 31, a sample containing the supernatant and the antioxidant was prepared before the heat retention in an oil bath. The types and the corresponding dosages of antioxidants mixed with the supernatant are as shown in Table 3. One grams of starch was added to the sample, and freeze-dried, and the freeze-dried product was kept warm in an oil bath set at a temperature of 70 °C for 65 hours, then the freeze-dried product was dissolved in ethanol, and the content of the hydroxy-α-sanshool in the dissolved freeze-dried product was measured by HPLC to calculate the survival rate. The results are shown in Table 3.

### [Comparative Example 2]

The content of hydroxy-α-sanshool in the freeze-dried product was measured by HPLC to calculate the survival rate in the same manner as in Examples 34 to 39 except that no antioxidant was added. The results are shown in Table 3.

### [Comparative Examples 3 and 4]

In the same manner as in Examples 1 to 31, a supernatant was prepared, and one grams of starch was added to the supernatant and freeze-dried, after which a powdery antioxidant was added to obtain a powdery mixture. The obtained powdery mixture was kept warm in an oil bath set at a temperature of 70 °C for 65 hours, and then the powdery mixture was dissolved in an ethanol, and the content of the hydroxy-α-sanshool was measured by HPLC to calculate the survival rate. The results are shown in Table 3.

**[Table 3]**

| | *Antioxidant* | *Antioxidant* | | Survival rate (%) |
|---|---|---|---|---|
| | | Dosage (µmol/mL) | Molar ratio (*1) | |
| Example 34 | Sodium ascorbate | 100 | 40 | 88 |
| Example 35 | | 200 | 80 | 94 |
| Example 36 | Gallic acid | 11.8 | 4.7 | 89 |
| Example 37 | | 23.5 | 9.4 | 94 |
| Comparative Example 2 | - | - | - | 39 |
| Comparative Example 3 | Sodium ascorbate | 100 | 40 | 38 |
| Comparative Example 4 | | 200 | 80 | 42 |

| | | | | |
|---|---|---|---|---|
| *1 The molar ratio of the antioxidant to hydroxy-alfa-sanshool | | | | |

It should be understood that the presently disclosed embodiments and examples are not more than illustrative in all respects and are non-limiting. It is intended that the scope of the invention be defined by the appended claims rather than by the foregoing embodiments and that all changes within the meaning and range of equivalency of the claims may be embraced therein.

### [Industrial applicability]

The sanshool stabilized by the stabilization method of sanshool of the present invention can be mixed with various products such as foods, supplements, beverages, pharmaceutical preparations, quasi-drugs, cosmetic preparations, feeds and the like.

## Claims

1. A method for stabilizing sanshool, the method comprises a mixing step of mixing an amount of sanshool with an amount of antioxidant in a liquid, wherein the liquid contains at least one selected from the group consisting of water and a water-soluble organic solvent, and the mixing step is performed under the condition of pH 6 or more.

2. The method for stabilizing sanshool according to Claim 1, wherein the antioxidant is one or more selected from the group consisting of dihydroxybenzoic acid or salt thereof or ester thereof, trihydroxybenzoic acid or salt thereof or ester thereof, hydroxycinnamic acid or salt thereof, ascorbic acid or salt thereof or ester thereof, and tocopherol.
